(19) Europäisches Patentamt
European Patent Office
Office européen des brevets

(11) Numéro de publication : 0 467 747 B1

(12) **FASCICULE DE BREVET EUROPEEN**

(45) Date de publication du fascicule du brevet : **10.11.93 Bulletin 93/45**

(51) Int. Cl.$^5$ : **C09B 67/50,** C09B 47/04, C07D 487/22, A61K 49/00

(21) Numéro de dépôt : **91401886.6**

(22) Date de dépôt : **08.07.91**

(54) **Nouveaux cristaux de phtalocyanine de lithium radicalaire, leur procédé de préparation et leur utilisation pour le dosage "in vivo" de l'oxygène moléculaire.**

(30) Priorité : **10.07.90 FR 9008739**

(43) Date de publication de la demande : **22.01.92 Bulletin 92/04**

(45) Mention de la délivrance du brevet : **10.11.93 Bulletin 93/45**

(84) Etats contractants désignés : **CH DE GB LI SE**

(56) Documents cités :
**EP-A- 0 352 182**
**FR-M- 2 181**

(56) Documents cités :
**JOURNAL OF MAGNETIC RESONANCE vol. 85, 1989, ORLANDO, US pages 50 - 59; R.K. WOODS ET AL.: 'spectral-spatial esr imaging as a method of noninvasive biological oxymetry '**
**J.chem.Soc.chem.Commu.1986,962-3-Sugimoto et al.**

(73) Titulaire : **COMMISSARIAT A L'ENERGIE ATOMIQUE**
**31-33, rue de la Fédération**
**F-75015 Paris (FR)**

(72) Inventeur : **Moussavi, Mehdi**
**20 rue Joseph Rolland**
**F-38120 Saint Egreve (FR)**

(74) Mandataire : **Mongrédien, André et al**
**c/o BREVATOME 25, rue de Ponthieu**
**F-75008 Paris (FR)**

EP 0 467 747 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux cristaux de phtalocyanine de lithium radicalaire.

De façon plus précise, elle concerne des cristaux de phtalocyanine de lithium radicalaire présentant des propriétés améliorées par rapport aux cristaux existants de phtalocyanine de lithium radicalaire, qui conviennent pour doser l'oxygène moléculaire au moyen d'un spectromètre à résonance paramagnétique électronique (RPE) dans de bonnes conditions.

Récemment, on a développé des procédés de mesure de la concentration en oxygène moléculaire des cellules par résonance paramagnétique électronique (RPE). Dans ces procédés, on utilise un traceur radicalaire dont les caractéristiques RPE (en premier lieu, la largeur de raie) varient notablement en fonction de la teneur en oxygène du milieu dans lequel se trouve le traceur radicalaire. Des techniques de dosage de l'oxygène moléculaire par spectrométrie RPE sont décrites par exemple dans les documents suivants : Swartz, Pure & Appl. Chem., vol. 62, n° 2, p. 235-239, 1990 ; Woods et al, Journal of Magnetic Resonance, n° 85, p. 50-59, 1989.

Pour mesurer la concentration d'oxygène intracellulaire par cette technique, on injecte ou on implante dans le milieu ou l'organe dont on veut connaître la teneur en oxygène, une substance radicalaire, puis on examine cette substance pendant qu'elle est immergée dans ce milieu au moyen d'un spectromètre à résonance paramagnétique électronique. A partir de la largeur de raie RPE obtenue, on en déduit la teneur en oxygène du milieu en se référant à une courbe d'étalonnage préalable d'un échantillon de la substance radicalaire donnant la relation entre la largeur de raie RPE et la teneur en oxygène.

Les substances radicalaires utilisées dans de tels procédés doivent présenter différentes propriétés. Ainsi, elles doivent être biocompatibles et avoir des caractéristiques RPE qui changent notablement en fonction de la teneur en oxygène du milieu, au moins dans la gamme des concentrations à suivre, qui est de 0 à 10% d'oxygène moléculaire dans le cas des organes humains qu'il est intéressant d'examiner.

Jusqu'à présent, on a utilisé comme substance radicalaire dans de tels procédés, la 2,2,6,6,-tétraméthylépipéridine-N-oxyl-4-one (TANO). Cependant, il serait intéressant de disposer d'autres substances radicalaires pour ces procédés car les variations des caractéristiques RPE du TANO dans la gamme de concentrations en oxygène la plus intéressante (0 à 10% d'oxygène) sont insuffisantes.

La présente invention a précisément pour objet une substance radicalaire utilisable pour le dosage de l'oxygène "in vivo" par résonance paramagnétique électronique, qui présente des caractéristiques plus intéressantes que celles du TANO dans la gamme de concentrations allant de 0 à 10%.

Cette substance radicalaire est constituée de cristaux de phtalocyanine de lithium radicalaire répondant à la formule :

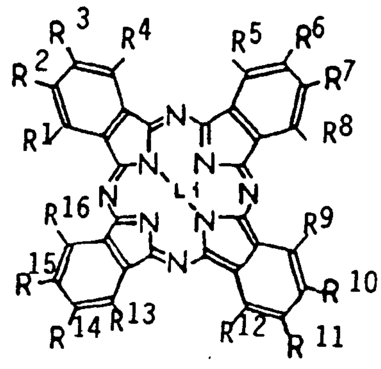

(I)

dans laquelle les $R^1$à $R^{16}$ qui peuvent être identiques ou différents, représentent un atome d'hydrogène ou de deutérium, et présentant une structure cristalline tétragonale appartenant au groupe spatial P4/mcc.

Selon un premier mode de réalisation de l'invention les $R^1$ à $R^{16}$ représentent tous un atome d'hydrogène.

Dans ce cas, les paramètres cristallographiques de la phtalocyanine sont les suivants :

a : 13,850 Å
b : 13,850 Å
c : 6,403 Å
$\alpha$ : 90°
$\beta$ : 90°
$\gamma$ : 90 °

volume de la maille : 1228 Å$^3$.

La phtalocyanine de lithium radicalaire de formule (I) dans laquelle $R^1$ à $R^{16}$ sont des atomes d'hydrogène est une substance radicalaire connue depuis longtemps, mais jusqu'à présent, on ne l'a jamais obtenue sous la forme de cristaux de structure tétragonale appartenant au groupe spatial P4/mcc.

En effet, les cristaux des phtalocyanines de lithium radicalaire connues ont une structure tétragonale appartenant au groupe spatial P4/nnc. Sugimoto et al., J. Chem. Soc., Chem. Commun., p 962-3, 1986 Or, cette différence est importante car la structure cristalline particulière (tétragonale, groupe spatial P4/mcc) de la phtalocyanine de lithium radicalaire de l'invention lui confère des caractéristiques RPE beaucoup plus intéressantes pour le dosage de l'oxygène moléculaire.

En effet, la largeur de raie RPE de la phtalocyanine de lithium radicalaire de l'invention est seulement de 2 000nT alors qu'elle est de 30 000nT dans le cas de la phtalocyanine de lithium radicalaire sous forme tétragonale, appartenant au groupe spatial P4/nnc.

Par ailleurs, avec la phtalocyanine radicalaire de l'invention la largeur de raie RPE varie pratiquement de façon linéaire avec la teneur en oxygène, dans la gamme de concentrations en oxygène allant de 0 à 10%, alors que la largeur de la raie RPE de la phtalocyanine de l'art antérieur ne varie pratiquement pas avec la teneur en oxygène.

Grâce à ces caractéristiques, on peut obtenir avec les cristaux de phtalocyanine de l'invention une meilleure précision sur la mesure des faibles taux d'oxygène dans les tissus biologiques. De plus, la forte sensibilité des cristaux à l'oxygène permet, soit d'injecter des quantités plus faibles de substance radicalaire si cela s'avère nécessaire, soit de diminuer le temps d'acquisition pour les courbes RPE et obtenir ainsi une réponse plus rapide sur les métabolismes d'organes.

Selon un second mode de réalisation de l'invention, la phtalocyanine de lithium de formule (I) est substituée au moins en partie par des atomes de deutérium.

Dans ce cas, on obtient des résultats encore meilleurs grâce à la présence des atomes de deutérium.

Généralement, la substitution porte sur au moins l'une des positions $R^1$, $R^4$, $R^5$, $R^8$, $R^9$, $R^{12}$, $R^{13}$ et $R^{16}$ de la formule (I) donnée ci-dessus car il est plus difficile de substituer par du deutérium les atomes d'hydrogène en position $R^2$, $R^3$, $R^6$, $R^7$, $R^{10}$, $R^{11}$, $R^{14}$ et $R^{15}$.

Les cristaux de phtalocyanine de lithium radicalaire répondant à la formule (I) donnée ci-dessus peuvent être obtenus par un procédé consistant à oxyder monoélectroniquement par voie galvanostatique de la phtalocyanine de lithium dilithiée répondant à la formule

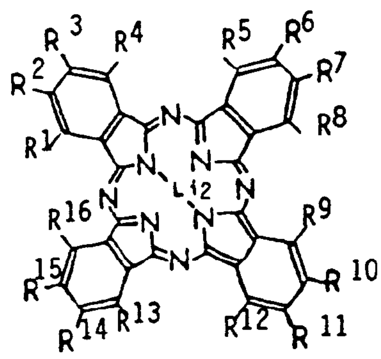

(II)

dans laquelle $R^1$ à $R^{16}$ ont la signification donnée ci-dessus,

en solution dans l'acètonitrile contenant au plus 50ppm d'eau, en utilisant une anode constituée par une plaque de platine, une cathode constituée par un fil de platine et un électrolyte-support constitué par de l'hexafluorophosphate ou du perchlorate de tétrabutylammonium.

Dans ce procédé, on réalise l'oxydation selon un mode galvanostatique, c'est-à-dire à courant constant, mais on peut opérer par paliers en effectuant plusieurs étapes à courant constant mais avec des intensités de courant différentes d'une étape à l'autre. Ainsi, on peut maîtriser la cinétique de croissance cristalline ou faire évoluer le système d'état d'équilibre en état d'équilibre lorsqu'on réalise plusieurs paliers et obtenir la forme cristalline recherchée.

Habituellement, on préparait la phtalocyanine de lithium radicalaire en oxydant monoélectroniquement la phtalocyanine de lithium dilithiée, mais en utilisant le mode potentiostatique, c'est-à-dire à potentiel constant, ce qui permet d'avoir un bon rendement. Dans ce cas, la cinétique de l'électrolyse n'est pas maîtrisée et l'on obtient une poudre en cristaux mal définis et mal formés qui ne correspondent pas aux aiguilles cristallines de l'invention.

Pour obtenir cette forme cristalline particulière, il est nécessaire de plus de choisir un solvant, un électrolyte support et des électrodes appropriés.

Comme on l'a indiqué ci-dessus, le solvant qui convient est l'acétonitrile contenant au plus 50ppm d'eau, qui a été de préférence purifié des traces d'acide acétique qu'il contient, par exemple par passage sur une colonne d'alumine.

En effet, si l'on opère avec un autre solvant tel que l'acétone, on n'obtient pas la structure cristalline voulue.

Les électrodes qui conviennent sont des électrodes en platine, et l'électrolyte-support peut être de l'hexafluorophosphate (ou du perchlorate) de tétrabutylammonium.

Par ailleurs, il est préférable que la cellule d'électrolyse ait la forme d'une demi-sphère.

Dans le cas où on veut obtenir des cristaux de phtalocyanine de lithium radicalaire deutérée, on opère de la même façon mais en partant de phtalocyanine de lithium dilithiée, deutérée.

La phtalocyanine de lithium dilithiée, deutérée peut être obtenue par échange isotopique H-D entre de la phtalocyanine dilithiée et de la tétraméthylpyridine deutérée, en présence d'un catalyseur à base de tétraméthylpyridine deutérée lithiée.

Les cristaux de phtalocyanine de lithium radicalaire de l'invention peuvent être utilisés en particulier pour le dosage d'oxygène moléculaire.

Aussi, l'invention a également pour objet un procédé de dosage de l'oxygène moléculaire dans un milieu tel qu'un liquide ou un gaz, qui consiste à mettre en contact ledit milieu avec au moins un cristal de phtalocyanine de lithium radicalaire de l'invention, et à déterminer la largeur de la raie de résonance paramagnétique électronique (RPE) de ce cristal.

Les phtalocyanines de lithium radicalaire de l'invention peuvent ainsi être utilisées pour le dosage "in vivo" de l'oxygène moléculaire, par exemple dans le coeur ou dans d'autres organes, en vue de détecter les cellules malades de ces organes, c'est-à-dire celles ayant des teneurs en oxygène inférieures à la normale.

Pour réaliser de tels examens "in vivo", on peut implanter des cristaux de la phtalocyanine radicalaire de l'invention directement dans l'organe à examiner, ou utiliser une suspension de ces cristaux dans un liquide organique que l'on injecte au patient à examiner.

Les liquides utilisés pour préparer de telles suspensions sont des liquides biocompatibles, non toxiques, par exemple les alcools tels que l'alcool furfurylique, les alcools en $C_4$ à $C_{10}$ et les polyalcools. On peut aussi utiliser du vératrol.

La suspension peut comprendre d'autres additifs si nécessaire, tels que des agents stabilisant la suspension ou réglant sa viscosité à une valeur appropriée.

La concentration en phtalocyanine de la suspension peut varier dans un large intervalle ; elle se situe de préférence dans la gamme allant de 0,1 à 10mg/l.

Bien que les phtalocyanines de lithium radicalaire de l'invention ne soient pas toxiques, les doses administrées sont généralement très faibles et peuvent aller par exemple de 0,01 à 0,1mg/kg de poids corporel.

Après implantation des cristaux ou administration de la suspension, on peut réaliser l'examen par spectrométrie RPE rapidement.

D'autres caractéristiques et avantages de l'invention apparaîtront mieux à la lecture de la description qui suit donnée bien entendu à titre illustratif et non limitatif, en référence au dessin annexé sur lequel :

- la figure 1 représente de façon schématique une cellule d'électrolyse utilisable pour préparer les cristaux de l'invention,
- la figure 2 représente de façon schématique un dispositif convenant à la réalisation de la courbe d'étalonnage de la substance radicalaire utilisée,
- la figure 3 est un diagramme représentant les variations de la largeur de raie RPE en fonction de la teneur en oxygène pour la phtalocyanine de lithium de l'invention (PcLi) et pour une substance radicalaire utilisée dans l'art antérieur (TANO), et
- la figure 4 est un diagramme représentant les variations de la raie de résonance RPE de la phtalocyanine de lithium au cours d'une expérience sur le rat.

Sur la figure 1, on a représenté de façon schématique une cellule d'électrolyse utilisable pour préparer les cristaux de phtalocyanine de lithium radicalaire de l'invention. Cette cellule (1) a une forme semisphérique et elle est fermée de façon étanche par un couvercle (3). A l'intérieur de la cellule, sont disposées une cathode (5) constituée par un fil métallique de platine ayant par exemple un diamètre de 1mm et une longueur de 4cm, et une anode (7) constituée par une plaque de platine de 25x35mm. L'anode et la cathode sont reliées à un générateur de courant électrique muni d'un dispositif approprié permettant de faire fonctionner la cellule selon le mode intensiostatique ou galvanostatique.

**Exemple 1** : Préparation des cristaux de phtalocyanine de lithium de l'invention.

On introduit dans la cellule (1) 300mg de phtalocyanine dilithiée en solution dans 500ml d'acétonitrile de qualité ultra anhydre. L'acétonitrile utilisé a une teneur en eau inférieure à 50ppm et il a été passé juste avant l'utilisation sur une colonne de poudre d'alumine basique d'activité I ayant un diamètre de 5cm et une hauteur de 10cm, pour éliminer les traces d'acide acétique qu'il contient éventuellement. On introduit également dans la cellule 400mg d'un électrolyte-support constitué par de l'hexafluorophosphate de tétrabutylammonium qui a été recristallisé 5 fois dans l'éthanol et séché pendant 24h sous vide à 80°C.

Après avoir fermé la cellule d'électrolyse, on réalise l'électrosynthèse en utilisant un courant constant de 5µA pendant 24h, puis un courant constant de 20µA pendant 48h et un courant constant de 50µA pendant 72h.

On recueille ainsi en fin d'opération, 150mg de phtalocyanine de lithium radicalaire sous forme d'aiguilles monocristallines noires qui présentent les caractéristiques cristallines données dans le tableau 1.

**Exemple 2.**

Dans cet exemple, on détermine les caractéristiques RPE des cristaux de l'exemple 1 en présence de concentrations d'oxygène différentes pour obtenir la courbe d'étalonnage correspondant aux variations de la largeur de raie RPE en fonction de la teneur en oxygène du milieu.

Dans ce but, on introduit dans un tube les cristaux de phtalocyanine de lithium obtenus précédemment, puis on fait le vide dans le tube et on mesure la largeur de la raie RPE après avoir introduit le tube maintenu sous vide dans un spectromètre RPE.

Ensuite, on refait les mêmes mesures après avoir mis en contact les cristaux avec des quantités différentes d'oxygène.

Ceci peut être effectué en utilisant le dispositif représenté de façon schématique sur la figure 2. Ce dispositif comprend une rampe à vide (11) munie d'un groupe de pompage (13) pour faire le vide dans la rampe qui peut être raccordée par l'intermédiaire d'une vanne (15) avec un tube (17) contenant les cristaux de phtalocyanine de lithium et par une vanne (19) avec un récipient (21) contenant un volume donné d'oxygène.

Pour effectuer la première mesure, on ouvre la vanne (15) après avoir disposé le tube (17) sur l'appareil et on fait le vide avec le groupe de pompage (13), puis on referme la vanne (15) et l'on introduit le tube (17) obturé dans le spectromètre RPE. Après cette mesure, on dispose à nouveau le tube (17) sur la rampe à vide (11), puis on ouvre la vanne (15) et la vanne (19) pour mettre en contact la phtalocyanine de lithium avec le volume donné d'oxygène du récipient (21). Après cette mise en contact, on ferme la vanne (15) et on introduit le tube (17) dans le spectromètre RPE pour avoir la largeur de raie à 9GHz.

Après cette opération, on dégaze à nouveau la phtalocyanine de lithium en disposant le tube (17) sur la rampe à vide (11) et en faisant le vide dans l'installation, puis on met ensuite en contact la phtalocyanine dégazée avec un autre volume d'oxygène en utilisant un autre récipient (21). On obtient ainsi les valeurs de largeur de raie RPE en fonction de la concentration en oxygène.

Sur la figure 3, on a représenté en trait plein cette courbe de variations de la largeur de de raie RPE (en $10^{-7}$T) en fonction de la teneur en oxygène (en %).

Sur cette figure, on voit que la variation de la largeur de raie est pratiquement linéaire dans le domaine de concentrations allant de 0 à 10% et que l'on peut détecter des variations très faibles de la teneur en oxygène dans cette gamme de concentrations.

Sur cette figure, on a représenté à titre comparatif (en tirets), les variations de largeur de raie RPE obtenues dans les mêmes conditions avec le TANO qui était précédemment utilisé pour le dosage d'oxygène par RPE.

Si l'on compare ces deux courbes, on remarque que le TANO ne permet pas de détecter des variations de concentration d'oxygène dans le domaine allant de 0 à 6%, ce qui montre bien l'intérêt de l'invention.

**Exemple comparatif 1.**

Dans cet exemple, on prépare de la phtalocyanine de lithium radicalaire à partir de la même phtalocyanine de lithium dilithié que celle utilisée dans l'exemple 1, mais on réalise l'oxydation électrochimique par voie potentiostatique dans de l'acétone à +0,5V. On obtient ainsi des cristaux de phtalocyanine de forme tétragonale appartenant au groupe spatial P4/nnc ayant les caractéristiques cristallographiques données dans le tableau 1.

On détermine ensuite la largeur de raie RPE de cette phtalocyanine de lithium en opérant dans les mêmes conditions que celles de l'exemple 1. On trouve que la largeur de raie est égale à 30 000nT, ce qui est nettement supérieur à ce que l'on obtient avec la phtalocyanine de lithium de l'invention. De plus, cette largeur de raie ne varie pratiquement pas avec le taux d'oxygène du milieu.

Ainsi, la phtalocyanine de lithium de l'art antérieur ne peut être utilisée pour doser l'oxygène moléculaire.

**Exemple 3.**

Dans cet exemple, on utilise les cristaux de phtalocyanine de lithium obtenus dans l'exemple 1 pour examiner l'évolution du taux d'oxygène dans un coeur de rat.

Dans ce but, on anesthésie le rat et on implante dans le coeur du rat un cristal de la phtalocyanine de lithium radicalaire de l'exemple 1.

On sacrifie ensuite le rat, puis on prélève son coeur et on l'introduit dans un spectromètre à résonance paramagnétique électronique.

On examine le signal obtenu juste après l'introduction du coeur dans le spectromètre à 1GHz, puis on relève le signal RPE obtenu après 6 minutes, puis les signaux obtenus toutes les trois minutes.

Les résultats obtenus sont représentés sur la figure 4.

Sur cette figure, la courbe 31 se réfère au signal obtenu au temps 0, la courbe 32 se réfère au signal obtenu après 6min, et les courbes 33, 34, 35, 36 et 37 se réfèrent respectivement aux signaux obtenus après 9min, 12min, 14min, 17min et 20min.

On remarque d'après ces résultats que la concentration en oxygène qui était de 17,2% au début, tombe à 1,5% au bout de 20min et que ces variations peuvent être facilement suivies par l'examen du signal RPE.

Les cristaux de phtalocyanine de lithium de l'invention peuvent donc être utilisés pour le dosage de l'oxygène intracellulaire.

L'exemple qui suit illustre la préparation d'une suspension de ces cristaux utilisable pour le dosage de l'oxygène chez les êtres vivants.

**Exemple 4.**

On introduit 1mg des cristaux de phtalocyanine de lithium radicalaire obtenu dans l'exemple 1 dans un tube en verre contenant 10ml d'hexanol. On congèle ensuite le contenu du tube dans l'azote liquide, on dégaze par pompage sous vide secondaire ($10^{-4}$Pa) pendant une demi-heure et on scelle ensuite le tube. Pour rendre le mélange homogène, on introduit le tube scellé dans une cuve à ultrasons pendant 15min.

Lors de l'utilisation, on ouvre le tube sous atmosphère inerte, par exemple dans une boite à gants et on prélève à l'aide d'une seringue étanche 0,1 à 0,5µl de la suspension prête à injecter dans l'organe à étudier.

Selon une variante, on peut utiliser des flacons à embout sertisable semblables aux flacons pharmaceutiques de soluté injectable qui sont fermés par une capsule d'aluminium munie d'un joint d'étanchéité en caoutchouc. Dans ce cas, on peut réaliser le dégazage par bullage d'argon dans le flacon à un débit de 4l/h pendant 20min pour 10ml de suspension.

## TABLEAU 1

| Substance radicalaire | PcLi (ex.1) | PcLi (ex. comparatif 1) |
|---|---|---|
| (largeur de raie RPE) | 2 000 nT | 30 000 nT |
| groupe spatial | P4/mcc tétragonal | P4/nnc tétragonal |
| a (Å) | 13,850 | 19,576 |
| b (Å) | 13,850 | 19,576 |
| c (Å) | 6,403 | 6,407 |
| α (degrés) | 90 | 90 |
| β (degrés) | 90 | 90 |
| γ (degrés) | 90 | 90 |
| volume de la maille (Å$^3$) | 1228 | 2455 |

## Revendications

1. Cristaux de phtalocyanine de lithium radicalaire répondant à la formule :

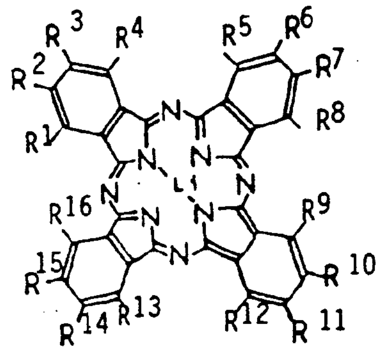

(I)

dans laquelle les $R^1$ à $R^{16}$ qui peuvent être identiques ou différents, représentent un atome d'hydrogène ou de deutérium, et présentant une structure cristalline tétragonale appartenant au groupe spatial P4/mcc.

2. Cristaux de phtalocyanine radicalaire selon la revendication 1, caractérisés en ce que les $R^1$ à $R^{16}$ représentent un atome d'hydrogène.

3. Cristaux de phtalocyanine de lithium radicalaire selon la revendication 1, caractérisés en ce que $R^2$, $R^3$, $R^6$, $R^7$, $R^{10}$, $R^{11}$, $R^{14}$ et $R^{15}$ représentent un atome d'hydrogène et au moins l'un des $R^1$, $R^4$, $R^5$, $R^8$, $R^9$, $R^{12}$, $R^{13}$ et $R^{16}$ représente un atome de deutérium.

4. Cristaux de phtalocyanine de lithium radicalaire selon la revendication 2, caractérisés en ce que leurs paramètres cristallographiques sont les suivants :

| | |
|---|---|
| a : | 13,850 Å |
| b : | 13,850 Å |
| c : | 6,403 Å |
| $\alpha$ : | 90° |
| $\beta$ : | 90° |
| $\gamma$ : | 90° |

volume de la maille : 1228 Å$^3$

5. Procédé de préparation de cristaux de phtalocyanine de lithium radicalaire selon l'une quelconque des revendications 1 à 4, caractérisé en ce qu'il consiste à oxyder monoélectroniquement par voie galvanostatique de la phtalocyanine de lithium dilithiée de formule :

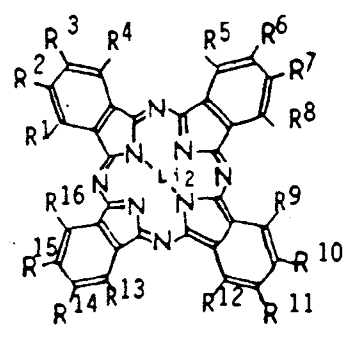

(II)

dans laquelle $R^1$ à $R^{16}$ ont la signification donnée dans la revendication 1, en solution dans l'acétonitrile contenant au plus 50ppm d'eau, en utilisant une anode constituée par une plaque de platine, une cathode constituée par un fil de platine et un électrolyte support constitué par de l'hexafluorophosphate ou du perchlorate de tétrabutylammonium.

6. Procédé de dosage de l'oxygène moléculaire dans un milieu, caractérisé en ce qu'il consiste à mettre en contact ledit milieu avec un cristal de phtalocyanine de lithium radicalaire selon l'une quelconque des revendications 1 à 4, et à déterminer la largeur de la raie de résonance paramagnétique électronique (RPE), de ce cristal de phtalocyanine de lithium radicalaire.

7. Composition pour le dosage "in vivo" de l'oxygène moléculaire, caractérisée en ce qu'elle est constituée par une suspension de cristaux de phtalocyanine de lithium radicalaire selon l'une quelconque des revendications 1 à 4, dans un liquide.

## Patentansprüche

1. Kristalle von radikalischem Lithiumphthalocyanin mit der Formel:

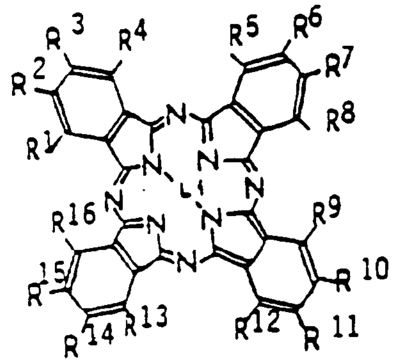

(I)

in welcher die $R^1$ bis $R^{16}$, die identisch oder voneinander verschieden sein können, ein Wasserstoffatom oder Deuteriumatom bedeuten und eine tetragonale Kristallstruktur, die der Raumgruppe P4/mcc angehört, aufweisen.

2. Kristalle von radikalischem Phthalocyanin nach Anspruch 1, **dadurch gekennzeichnet,** daß die $R^1$ bis $R^{16}$ ein Wasserstoffatom bedeuten.

3. Kristalle von radikalischem Lithiumphthalocyanin nach Anspruch 1, **dadurch gekennzeichnet,** daß $R^2$, $R^3$, $R^6$, $R^7$, $R^{10}$, $R^{11}$, $R^{14}$ und $R^{15}$ ein Wasserstoffatom bedeuten und mindestens einer von $R^1$, $R^4$, $R^5$, $R^8$, $R^9$, $R^{12}$, $R^{13}$ und $R^{16}$ ein Deuteriumatom bedeutet.

4. Kristalle von radikalischem Lithiumphthalocyanin nach Anspruch 2, **dadurch gekennzeichnet,** daß ihre kristallographischen Parameter die folgenden sind:
   a : 13,850 Å
   b : 13,850 Å
   c : 6,403 Å
   $\alpha$ : 90°
   $\beta$ : 90°
   $\gamma$ : 90°
   Volumen der Elementarzelle: 1228 Å$^3$

5. Verfahren zur Herstellung von Kristallen von radikalischem Lithiumphthalocyanin nach einem der Ansprüche 1 bis 4, dadurch gekennzeichnet, daß es darin besteht, daß auf galvanostatischem Weg das zweifach lithiierte Lithiumphthalocyanin mit der Formel:

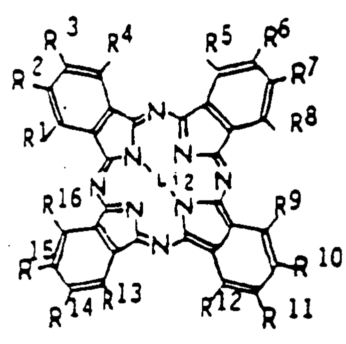

(II)

in welcher $R^1$ bis $R^{16}$ die in Anspruch 1 angegebene Bedeutung haben, in Lösung in Acetonitril, welches höchstens 50 ppm Wasser enthält, unter Verwendung einer Anode, die aus einer Platinplatte besteht, einer Kathode, die aus einem Platindraht besteht, und eines Trägerelektrolyts, der aus Tetrabutylammoniumhexafluorphosphat oder -perchlorat besteht, monoelektronisch oxidiert wird.

6. Verfahren zur Dosierung von molekularem Sauerstoff in einer Umgebung, **dadurch gekennzeichnet,** daß es darin besteht, die Umgebung mit einem Kristall von radikalischem Lithiumphthalocyanin nach einem der Ansprüche 1 bis 4 in Kontakt zu bringen und die Linienbreite der Elektronenspinresonanz (ESR) dieses Kristalls von radikalischem Lithiumphthalocyanin zu bestimmen.

7. Zusammensetzung zur Dosierung "in vivo" von molekularem Sauerstoff, **dadurch gekennzeichnet,** daß sie aus einer Suspension von Kristallen von radikalischem Lithiumphthalocyanin nach einem der Ansprüche 1 bis 4 in einer Flüssigkeit besteht.

**Claims**

1. Radical lithium phthalocyanine crystals according to the formula:

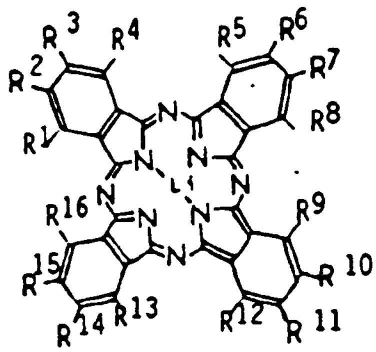

(I)

in which $R^1$ to $R^{16}$, which can be the same or different, represent a deuterium or hydrogen atom and have a tetragonal crystalline structure belonging to the space group P4/mcc.

2. Radical phthalocyanine crystals according to claim 1, characterized in that the $R^1$ to $R^{16}$ represent a hydrogen atom.

3. Radical lithium phthalocyanine crystals according to claim 1, characterized in that $R^2$, $R^3$, $R^6$, $R^7$, $R^{10}$, $R^{11}$, $R^{14}$ and $R^{15}$ represent a hydrogen atom and at least one of the $R^1$, $R^4$, $R^5$, $R^8$, $R^9$, $R^{12}$, $R^{13}$ and $R^{16}$ represents a deuterium atom.

4. Radical lithium phthalocyanine crystals according to claim 2, characterized in that their crystallographic parameters are as follows:

| | |
|---|---|
| a : | 13.850 Å |
| b : | 13.850 Å |
| c : | 6.403 Å |
| $\alpha$ : | 90° |
| $\beta$ : | 90° |
| $\gamma$ : | 90° |

mesh volume : 1228 Å$^3$

5. Process for the preparation of radical lithium phthalocyanine crystals according to any one of the claims 1 to 4, characterized in that it consists of galvanostatically monoelectronically oxidizing the dilithiated lithium phthalocyanine of formula:

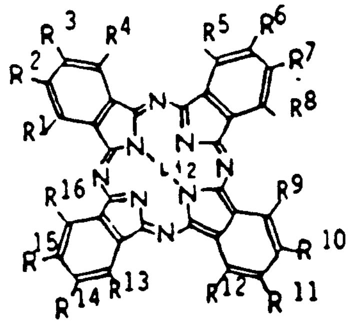

(II)

in which $R^1$ to $R^{16}$ have the meanings given in claim 1, in solution in acetonitrile containing at the most 50 ppm of water, using an anode constituted by a platinum plate, a cathode constituted by a platinum wire and a support electrolyte constituted by tetrabutyl ammonium perchlorate or hexafluorophosphate.

6. Process for determining molecular oxygen in a medium, characterized in that it consists of contacting the said medium with a radical lithium phthalocyanine crystal according to any one of the claims 1 to 4 and determining the electronic paramagnetic resonance (EPR) line width of said radical lithium phthalocyanine crystal.

7. Composition for the in vivo determination of molecular oxygen, characterized in that it is constituted by a suspension of radical lithium phthalocyanine crystals according to any one of the claims 1 to 4 in a liquid.

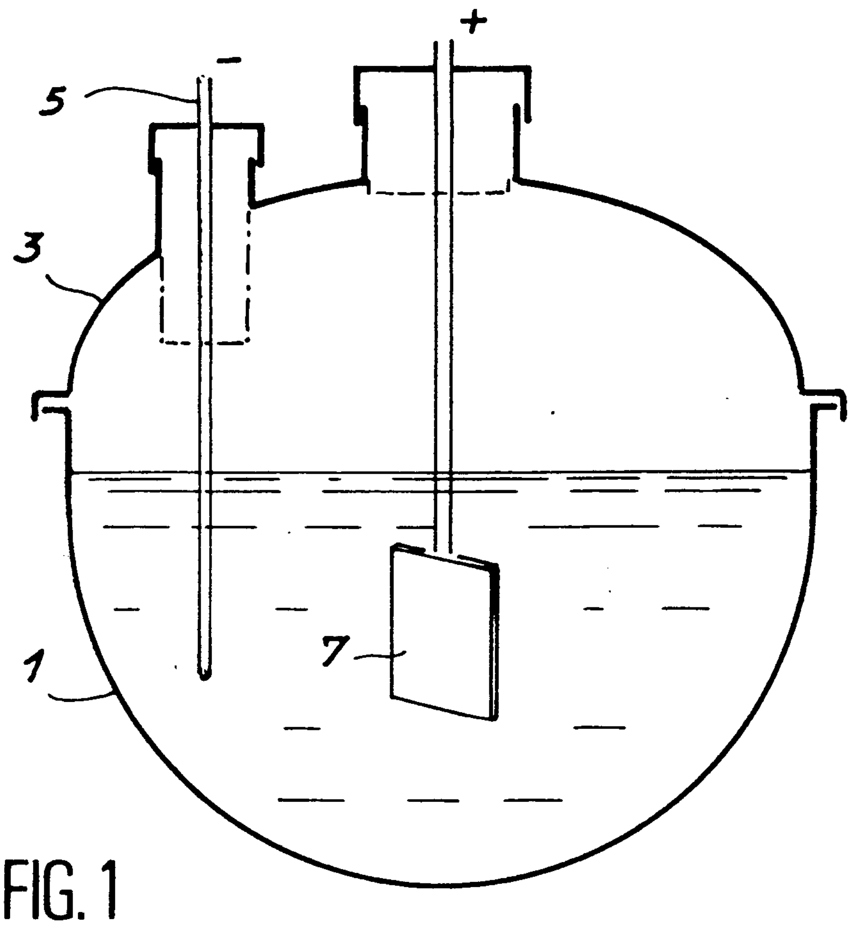


FIG. 1

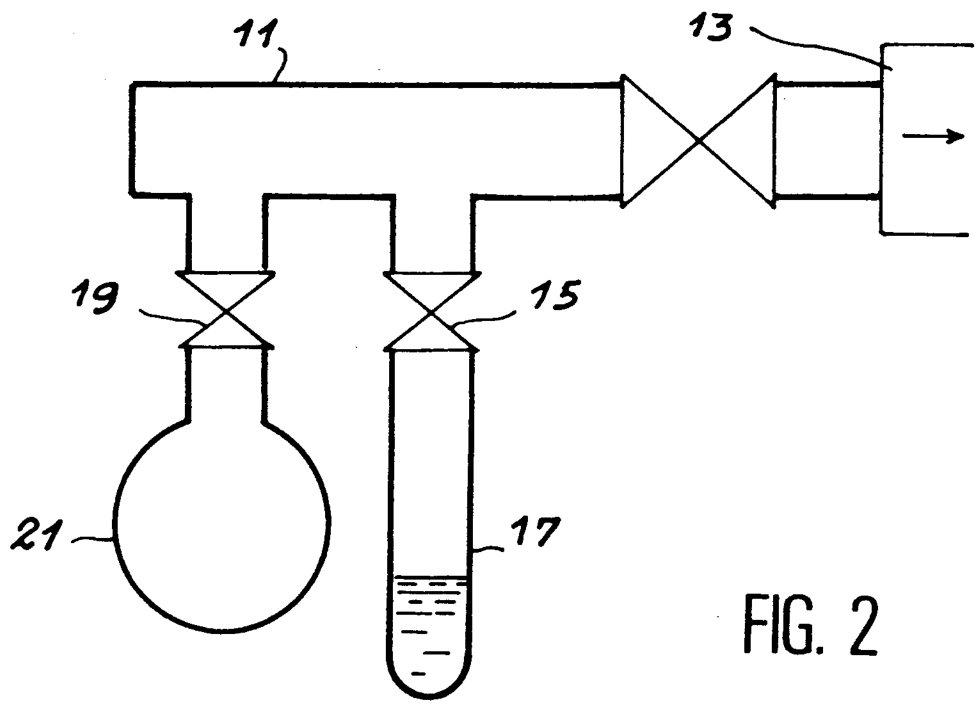


FIG. 2

FIG. 3

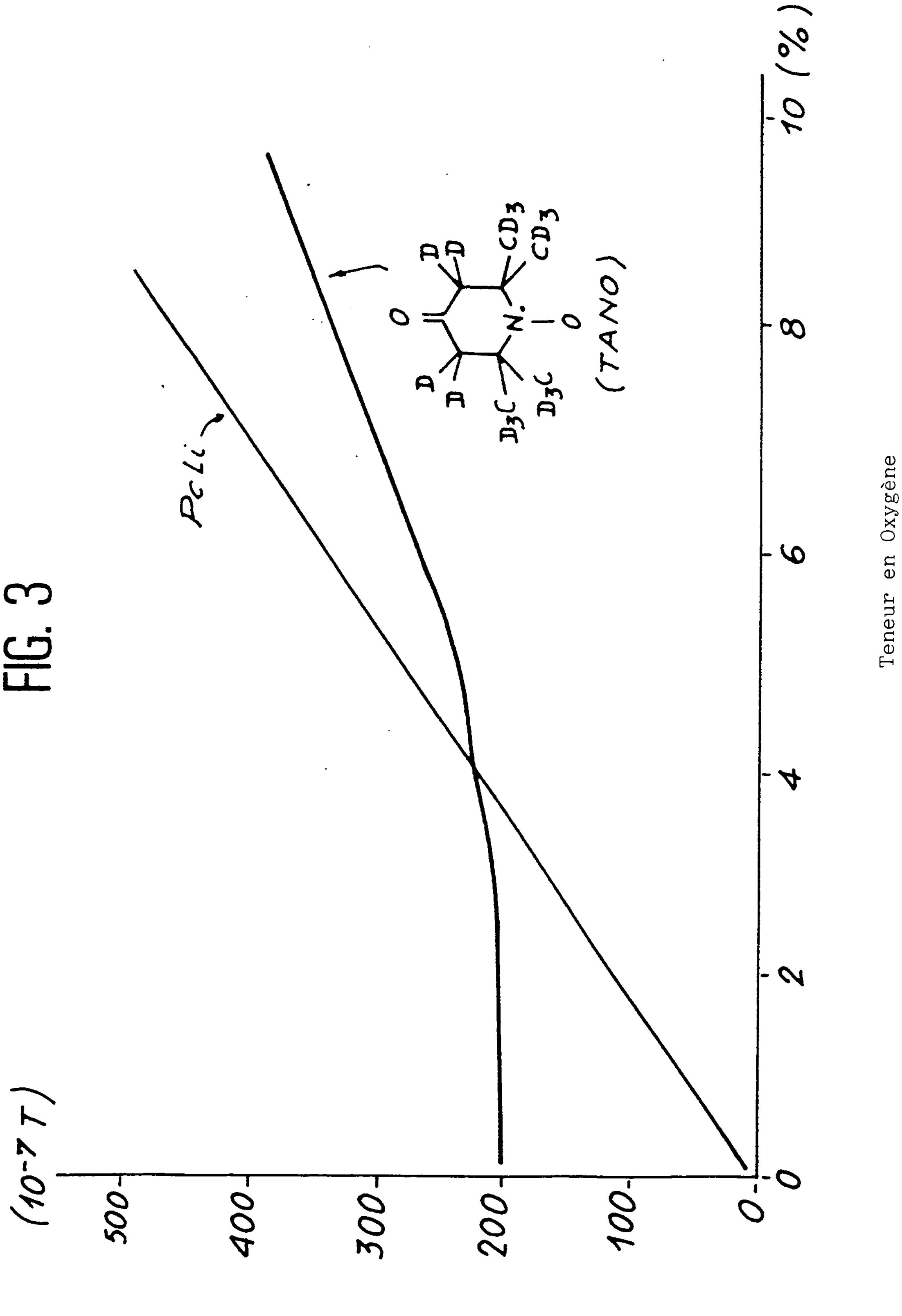
(10⁻⁷ T)
500
400
300
200
100
0
0
2
4
6
8
10
(%)
PcLi
(TANO)
Teneur en Oxygène

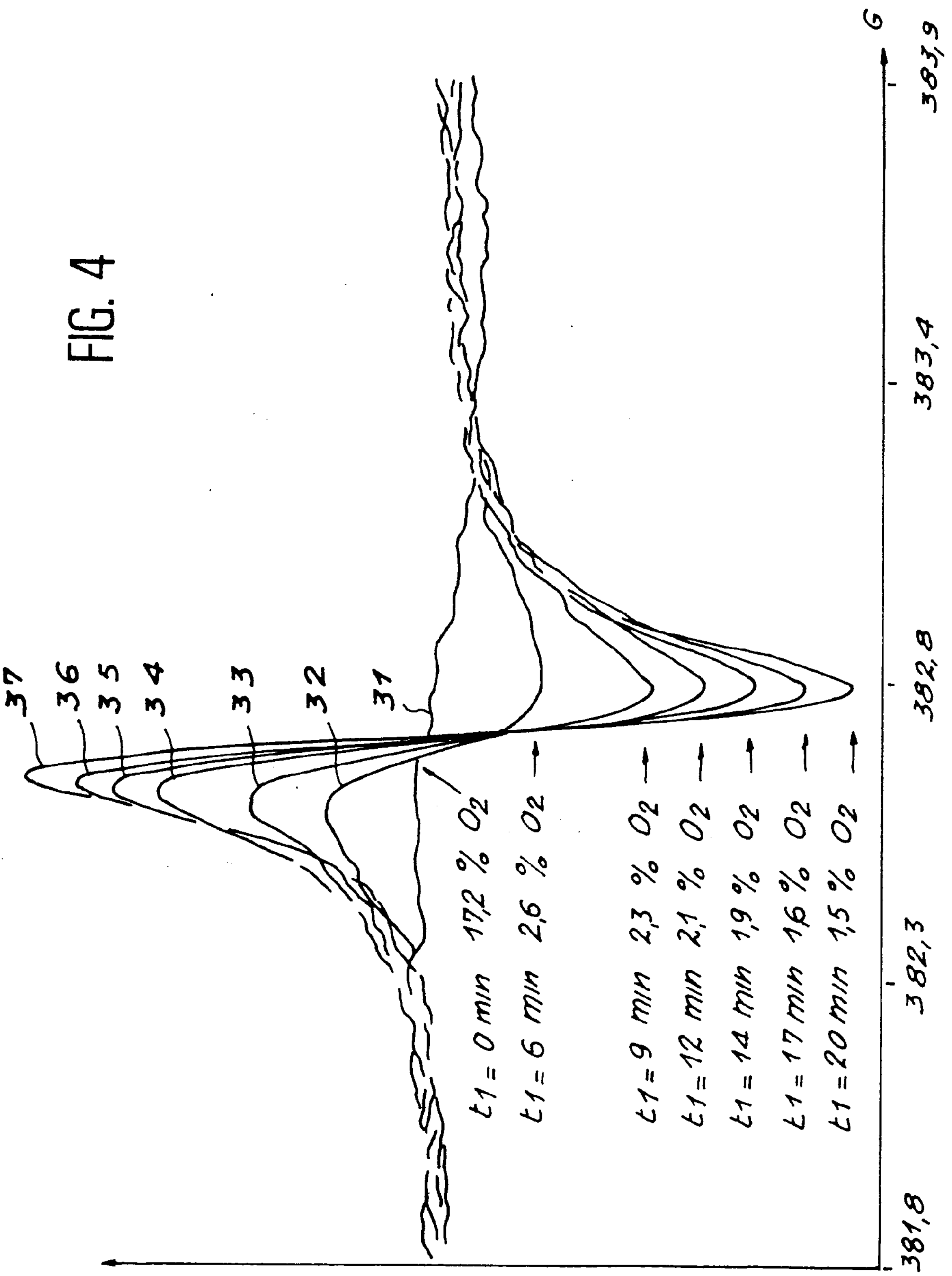
37
36
35
34
33
32
31
t1 = 0 min 17,2 % O2
t1 = 6 min 2,6 % O2
t1 = 9 min 2,3 % O2
t1 = 12 min 2,1 % O2
t1 = 14 min 1,9 % O2
t1 = 17 min 1,6 % O2
t1 = 20 min 1,5 % O2
381,8
382,3
382,8
383,4
383,9
G

FIG. 4